# EUROPÄISCHE PATENTSCHRIFT

(11) **EP 1 032 342 B1**
(45) Veröffentlichungstag und Bekanntmachung des Hinweises auf die Patenterteilung: **25.07.2001**
(21) Anmeldenummer: 98961201.5
(22) Anmeldetag: 16.11.1998
(51) Int. Cl.: A61F 13/15

(54) **VERFAHREN UND VORRICHTUNG ZUR HERSTELLUNG EINER AUS CELLULOSE-FASERN BESTEHENDEN FASERSTOFFBAHN FÜR DIE VERWENDUNG IN HYGIENEARTIKELN**
METHOD AND DEVICE FOR PRODUCING A STRIP OF CELLULOSE FIBRE MATERIAL FOR USE IN HYGIENE ARTICLES
PROCEDE ET DISPOSITIF POUR PRODUIRE UNE BANDE DE MATERIAU FIBREUX A BASE DE FIBRES DE CELLULOSE, S'UTILISANT DANS LE DOMAINE DES ARTICLES D'HYGIENE

(30) Priorität: 18.11.1997 DE 19750890; 04.06.1998 DE 19824825
(43) Veröffentlichungstag der Anmeldung: 06.09.2000
(73) Patentinhaber: Maksimow, Alexander, 48565 Steinfurt-Borghorst (DE)
(72) Erfinder: Maksimow, Alexander, 48565 Steinfurt-Borghorst (DE)
(74) Vertreter: Hoffmeister, Helmut, Dr. Dipl.-Phys.
(86) Internationale Anmeldenummer: EP9807305
(87) Internationale Veröffentlichungsnummer: WO9925281

(56) Entgegenhaltungen:
- WO-A-95/27429
- US-A- 3 692 622
- US-A- 4 749 423
- US-A- 5 128 193

## Beschreibung

Die Erfindung betrifft ein Verfahren gemäß Oberbegriff des Anspruchs 1 und eine Vorrichtung zur Herstellung einer aus Cellulose-Fasern bestehenden Faserstoffbahn gemäß den Oberbegriff des Anspruches 14 für die Verwendung in Hygieneartikeln, insbesondere persönliche, absorbierenden Hygieneartikeln. Ferner betrifft die Erfindung nach den Verfahren hergestellte absorbierende Faserstoffbahn.

Es ist bekannt, cellulosehaltiges Material, wie Holz- oder Pflanzenfasern, zu einer Faserstoffbahn zu verbinden, indem eine Kombination aus mechanischen und chemischen Verfahrensschritten unter intensiver Erhitzung verwendet wird, wobei unter Sauerstoffabschluß gearbeitet wird. Das Ziel eines derartigen Verfahrens ist es, wenige oder praktisch keine Bindemittel-Additive verwenden zu müssen. Nach einem dieser bekannten Verfahren (US A1 4,111,744) werden Cellulose-Fasern mit einer Feuchte von 3 bis 12 Gew.-% in einer sauerstofffreien Atmosphäre in einem Temperaturbereich von 450 bis 800 °F (= 232 bis 426 °C) einem Druck unterworfen, wobei eine hohe Umgebungstemperatur jenseits der Verkohlungstemperatur von Cellulose und der Zündtemperatur der Cellulose gegeben ist. Mit Hilfe des vorgenannten bekannten Verfahrens können auch papierähnliche Produkte, im allgemeinen jedoch nur eine Art steifer Karton, hergestellt werden.

Nachteil bei diesem Verfahren ist es, daß ein hoher technologischer Aufwand für die Beheizung der Druckbereiche und das Verhindern des Entzündens des Materials durch sauerstofffreie Fertigung getrieben werden muß.

Bekannt ist weiterhin ein Verfahren (WO 94/10956), um aus trockenen Cellulose-Fasern und Zusatzstoffen unter Druck absorbierende Bahnenware herzustellen, indem aus einem Material mit einem Flächengewicht von 30 bis 2000 g/cm² ein absorbierendes Produkt mit einer spezifischen Dichte von 0,2 bis 1,0 g/cm³ komprimiert wird. Das Komprimieren geschieht zwischen glatten Kalanderwalzen. Nachteilig bei diesem Verfahren ist, daß zwar eine Erhöhung der Dichte eintritt, jedoch das Material in sich wenig Reißfestigkeit besitzt. Um die Reißfestigkeit zu erhöhen, müssen synthetische Zusatzstoffe hinzugefügt werden, insbesondere Thermoplasten.

Weiterhin ist aus US-A 3 692 622 bekannt, zunächst eine regellose Zellstoff-Faserlage zu bilden und unter relativ niedrigem Druck ein lockeres Vlies mit geringer Dichte und Reißfestigkeit zu erzeugen. Das lockere Vlies wird in den Spalt eines weiteren Kalanderrollenpaares eingeführt und mit einem Muster von punkt- oder linienförmigen Druckbereichen geprägt. Es ergibt sich ein weiches, absorbierendes Bahnmaterial, das ein Grundgewicht von etwa 16,9 bis 50,9 g/m² hat. Die Reißfestigkeit dieser Faserbahn beträgt etwa 0,09 kN/m. Es handelt sich also um ein leicht reißendes Material, wie es z.B. bei einem Papiertaschentuch vorliegt. Die bei dem bekannten Produkt angewandten Kalander-Drücke liegen bei 2000 bis 10.000 p.s.i., entsprechend 14 bis 69 MPa. In der US-A wird davon gesprochen, daß sich eine Wasserstoffbrücken-Bindung ("hydrogen bonding') ergeben soll, wie es sich gewöhnlich in selbstbindenden konventionellen Papierprodukten ergibt.

Die nach dem Verfahren hergestellte Faserstoffbahn soll sich insbesondere zur Herstellung von Hygieneprodukten eignen. Sie soll hoch saugfähig, weich und als Bahn verarbeitet war sein. Hygieneartikeln zum Einmalgebrauch, wie Höschenwindeln und dgl., werden in großer Zahl hergestellt. Die für sie verwendeten absorbierenden Kernschichten sollen möglichst gut körperverträglich sein, die eintretende Flüssigkeit gut verteilt aufnehmen und nach Gebrauch auf entsprechenden Deponien rückstandslos verrotten. Es ist bekannt, die absorbierende Schicht aus einer Holzcellulosefaser-Matrix herzustellen, wobei dieser Fasermatrix sogenannte Superabsorber zur Erhöhung der Flüssigkeitsaufnahmekapazität hinzugefügt werden können. Superabsorber sind Polymere, die unter Bildung von Hydrogelen Wasser aufnehmen können.

Es stellt sich die Aufgabe, ein Verfahren zur Herstellung einer aus Cellulose-Fasern bestehenden Faserstoffbahn anzugeben, bei den praktisch keine Bindestoffe verwendet werden müssen, wobei bei Zimmertemperaturen und unter normalem Atmosphärendruck und Sauerstoffgehalt der Umgebungsluft gearbeitet werden kann.

Diese Aufgabe wird gelöst mit einem Verfahren zur Herstellung einer aus Cellulose-Fasern bestehenden, weitgehend reißfesten, saugfähigen und rollbaren Faserstoffbahn mit den Verfahrensschritten gemäß Kennzeichen des Anspruches 1.

Dabei wird davon ausgegangen, daß es in der Technologie der Herstellung von zu Cellulose-Fasern bekannt ist, diese aus einem Holzderivat mit der Fachbezeichnung "fluff pulp" herzustellen. Bei diesem Stoff handelt es sich um ein standardisiertes Produkt aus Holz, das aus in Platten oder Bahnen, sogenannten wood pulp cardboards, geliefertem Cellulosematerial hergestellt wird, das vor der Verwendung üblicherweise in Hammermühlen verkleinert und aufgefasert wird, bis ein watteähnliches Produkt aus Cellulose-Fasern, nämlich fluff pulp, entstanden ist. Eine Beschreibung eines solchen standardisierten Zerkleinerungsverfahrens findet sich beispielsweise in dem Prospekt der Firma Dan-Webforming International A/S, risskov, Dänemark.

Bei diesem "fluff pulp" genannten Holzderivat handelt es sich um ein Produkt, das insbesondere bei der sogenannten wasserlosen Papierfertigung in großen Mengen verwendet wird. Die Faser haben vorzugsweise in Länge etwa zwischen 1 und 5 mm, wenn sie aus der Hammermühle heraustreten. Sie liegen gemäß dem ersten Schritt des vorgenannten Verfahrens völlig regellos in einer Cellulose-Faserlage von etwa 5 bis 15 mm Höhe und werden vorzugsweise auf einem Förderband oder einem beweglichen Sieb durch eine erste Vorverdichtungsstation geschickt, die vorzugsweise aus einem Kalanderrollen-Paar mit geringem Druck besteht, so daß ein lockeres Vlies mit geringer Dichte und Reißfestigkeit entsteht. Die Reißfestigkeit ist so hoch bemessen, daß das Vlies über eine Länge von etwa 0,1 bis 1 m frei durchhängen kann ohne zu zerreißen. Es kann auch einem Luftdruck widerstehen, wie er bei der Fertigung auftritt.

Dieses an sich bekannte und noch sehr lockere Vlies wird in den Spalt eines Kalanderrollen-Paares eingeführt, wo ein Druck in den punktförmigen Drückbereichen von erheblicher Höhe aufgebracht wird. Der Druck muß mindestens 100 und sollte etwa 520 MPa = (MPa = mm²) betragen. Eine Grenze für den Druck nach oben bildet im allgemeinen die Fließgrenze des für die Walzen verwendeten Materials. Bisher wurde nach dem Stand der Technik nicht mit derartig hohen Drucken gearbeitet. Zur Erzeugung eines solchen Druckes können Walzen mit Noppen, verschränkt zueinander laufenden Linienmustern oder anderen überstehenden punkt- oder linienartigen Druckflächen vewendet werden, wobei die Rasterdichte der punktförmigen Druckbereiche zwischen 1 und 16 Rasterpunkte pro cm² liegt.

Nach dem Verfahren wird eine Faserstoffbahn mit vorzugsweise einem m²-Gewicht zwischen 50 g und 1500 g erzeugt. Die neuartige Faserstoffbahn ist durch die rasterförmige Verteilung der Verbindungspunkte so fest geworden, daß eine Reißfestigkeit von wenigstens 0,12 kN/m, vorzugsweise bis 0,65 kN/m, erreicht wird. Die Dicke der Faserstoffbahn richtet sich nach der gewünschten Metrage.

Die drückende Fläche der punktförmigen Druckbereiche bemißt sich danach, welche Drücke zwischen den zweiten Kalanderwalzen zu erreichen ist. Ausreichend haben sich punktförmige Druckbereiche mit einer Fläche zwischen 0,05 und 10 mm² ergeben.

Wie bereits betont, sollte die Temperatur des zweiten Kalanderrollen-Paares auf Zimmertemperatur, d.h. zwischen 18 und 25°C, gehalten werden. Es läßt sich auch bei höheren Temperaturen arbeiten. Zu bemerken ist auch, daß die Temperatur in den Druckbereichen aufgrund der erheblichen verbrauchten Leistung ansteigt.

Die Vorverdichtung sollte bei einer Werkzeugtemperatur erfolgen, die zwischen 18° und 320°C liegt, vorzugsweise zwischen 250 und 300°C. Als vorverdichtendes Werkzeug wird vorzugsweise ein erstes Kalanderrollen-Paar verwendet, das beheizbar ist.

Die Faser und/oder das lockere Vlies werden vor Eintritt in die Kalanderrollen vorzugsweise auf eine gewisse Feuchte gebracht, wobei vorzugsweise diese zwischen 2 und 9 Gew.-%- eingestellt werden sollte, wenigstens jedoch auf 1,5 Gew.%.

Als Ausgangsmaterial wird das bereits genannte fluff pulp-Holzderivat verwendet. Hierbei handelt es sich vorzugsweise um standardisierte defiberisierte Ware, wie sie in auch zur Herstellung von Faserstoffbahnen nach bekannten Verfahren eingesetzt wird. Als sehr vorteilhaft erscheint die Verwendung von sulfit- oder sulfat-gebleichten Langfaser-Celluloseen aus nördlichen Hölzern.

Vorteilhaft hat sich weiterhin erwiesen, wenn die Cellulose-Fasern nicht bis zur völligen Weiße gebleicht sind, sondern noch einen gewissen Anteil an natürlichen Holzstoffen enthalten. Dies zeigt sich in einem Weißegrad, der zwischen 80 bis 92 %, vorzugsweise von 85 bis 89 %, liegen sollte. Auch ein gewisser Restgehalt an Lignin ist vorteilhaft, wenn dieser beispielsweise zwischen 0,5 bis 5 Gew.-% des Ausgangsmaterials liegt.

Dem Ausgangsmaterial können auch nicht-bindende, anorganische Pigmente oder Füllstoffe, beispielsweise Titandioxid, Kaolin oder Zeolithe zugefügt werden.

Auch kann den Ausgangsfasern ein Anteil an Superabsorbern beigefügt werden, wobei die als Superabsorber bekannten Acrylatverbindungen sich in Pulverform in einem Anteil - bezogen auf die Gesamtmenge - von beispielsweise 0,5 bis 70 Gew.-% dem fluff pulp beimischen lassen, wobei das Herstellungsverfahren hierdurch nicht wesentlich beeinträchtigt wird.

Im Druckbereich des zweiten Kalanderrollen-Paares sollte der radiale Abstand der Kalanderrollen außerhalb der punktförmigen Druckbereiche etwa 1 bis 15 mm betragen, so daß sich das Material beim Druckvorgang außerhalb der Druckbereiche nicht quetscht, sondern vielmehr bauscht und etwas zusammenpreßt.

Der Spalt im Druckbereich des zweiten Kalanderrollen-Paares bemißt sich nach der Metrage und der Dicke des eingeführten lockeren Vlieses. Im allgemeinen sollte der Spalt eine lichte Weite von 0,05 bis 1 mm nicht überschreiten.

Wesentliches Teil der Vorrichtung zur Durchführung des Verfahrens bildet das zweite Kalanderrollen-Paar, das vorzugsweise aus zwei stählernen Kalanderwalzen besteht, die beide mit zahlreichen, über die Walzenmantelflächen verteilten Noppen, entsprechenden punktförmigen Druckbereichen oder Linienzügen versehen sind, die von Vertiefungen umgeben sind, die das Mehrfache des Volumens der erhöhten Bereiche aufweisen. Die erhöhten Bereiche stehen sich bei beiden Walzen im Arbeitsspalt gegenüber, wobei in punktförmigen Druckbereichen auf das zwischen den Druckbereichen befindliche lockere Vlies ein Druck von wenigstens 200 MPa bis maximal zur Fließgrenze des für die Noppen verwendeten Materials ausübbar ist.

Die Höhe der Noppen oder der anderen Druckbereiche beträgt vorzugsweise beträgt zwischen 0,5 und 15 mm gegenüber dem Walzengrund. Vorzugsweise haben die Noppen die Form von Pyramiden- oder Kegelstümpfen, deren Noppenmantel-Winkel gegenüber dem Radius zwischen 10 und 45° liegt. Auch linienförmige Druckbereiche oder dergleichen sind möglich.

Die regellos liegenden Fasern sind unter sehr hohem lokalen Druck in linien- oder punktförmigen Druckbereichen aufeinander gedrückt, so daß eine Vielzahl von innigen, sich nach Aufhebung des Druckes nicht-lösenden Fusionen der Faserkörper erfolgen. Es wird ein Produkt aus zahlreichen regellosen Cellulose-Fasern hergestellt, die an den Druckbereichen durch Faserverklebung verbunden sind. Die Faserstoffbahn hat eine ausreichend Reißfestigkeit und darüber hinaus eine hohe Absorptionsfähigkeit, so daß sie für Hygieneartikel hervorragend geeignet ist.

Es hat sich gezeigt, daß zur Erfüllung von spezifischen Anforderungen des Hygienesektors die Bahn aus Fasermaterial in arbeitsintensiver Weise nachträglich mit geeigneten Materialien kombiniert werden muß. Es stellt sich demnach die zusätzliche Aufgabe, ein Verfahren zur Herstellung einer aus Cellulose-Fasern bestehenden Faserstoffbahn anzugeben, das mit zusätzlichen Merkmalen, wie beispielsweise verstärkter Reißfestigkeit, Dichtigkeit oder Atmungs- und/oder Isolierfähigkeit ausgestattet ist.

Diese Aufgabe wird gelöst bei einem Verfahren gemäß den Ansprüchen 16 oder 17.

Die in den Ansprüchen genannten Verfahren werden anhand von Ausführungsbeispielen und anhand der Zeichnung erläutert, wobei die Figuren der Zeichnung zeigen:
- Fig. 1: in schematischer Darstellung eine Vorrichtung zur Herstellung einer aus Cellulose-Fasern bestehenden Faserstoffbahn;
- Fig. 2: in vergrößerter Darstellung gemäß Fig. 1 im Querschnitt den Druckbereich zweier Walzen mit pyramidenförmigen Noppen;
- Fig. 3: in perspektivischer Darstellung einen Abschnitt des nach dem Verfahren hergestellten, fertigen Produktes;
- Fig. 4: eine vergrößerte Darstellung der Druckbereiche der Faserbahn;
- Fig. 5: eine schematische Darstellung einer anderen Vorrichtung zur Herstellung einer Faserstoffbahn mit zwei zusätzlichen Kunststoffschichten;
- Fig. 6: eine schematische Darstellung einer weiteren Vorrichtung zur Herstellung einer Faserstoffbahn mit einer Kunststoffbeschichtung;
- Fig. 7: in einer Darstellung ähnlich wie Fig. 2 im Querschnitt den Druckbereich zweier Walzen mit dazwischenliegender Faserbahn mit aufliegender Folie;

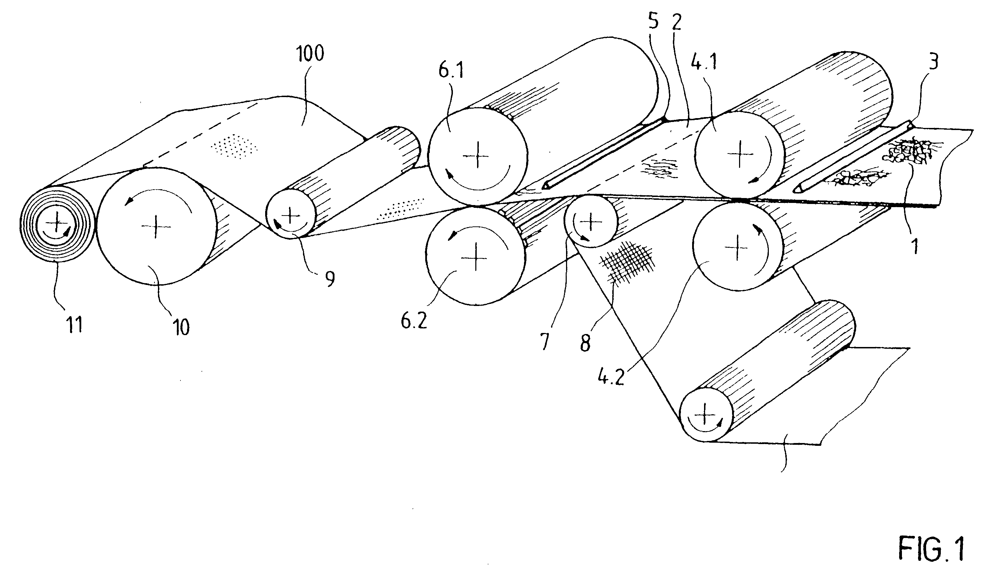

In Fig. 1 ist in schematischer Reihenfolge eine Anordnung von Walzen und Rollen dargestellt, mit denen das Verfahren durchgeführt wird. Der Herstellungsprozeß geht aus von Cellulose-Fasern, die als fluff pulp vorzugsweise aus trockenem wood pulp cardboards mit Hilfe von Hammermühlen hergestellt werden, was in dem Stand der Technik genannten Prospekt Dan Webforming International A/S sehr detailliert dargestellt ist.

Eine Schicht aus regellosen Fasern 1 von etwa 20 mm Höhe wird auf dem Siebförderband 8 zu einem ersten Kalanderwalzen-Paar 4.1, 4.2 gefördert. Die obere Walze 4.1 hat eine Oberflächentemperatur von etwa 220°C, während die untere unbeheizt ist. Vor Eintritt in den Spalt zwischen den beiden Walzen 4.1 und 4.2 wird die Bahn mit Hilfe einer Befeuchtungsvorrichtung 3 durch Besprühen von der Oberseite her befeuchtet, wobei die Feuchte des Materials danach etwa 5 bis 10 Gew.% ausmacht.

Zwischen den Kalanderwalzen 4.1 und 4.2, wird ein Teil der Feuchte wieder ausgetrieben und die regellose Cellulose-Faserlage zu einem lockerem Vlies mit geringer Dichte und Reißfestigkeit zusammengepreßt. Die Reißfestigkeit reicht aber aus, daß das Vlies 2 bei Überbrückung der Strecke zwischen dem Ende des Siebbandes 8 an der Umlenkrolle 7 bis zum Eintritt in einen Spalt zwischen zwei weiteren Kalanderrollen 6.1 und 6.2, die etwa 50 cm beträgt, nicht abreißt.

Der erste Verfahrensschritt stellt lediglich ein Vorverdichen oder Kompaktieren des Vlieses aus den regellos liegenden Fasern dar. Eine feste Bahn wird nicht gebildet, und es ist ohne weiteres möglich, die Fasern einzeln Stück für Stück zu entnehmen. Die Reißfestigkeit des Vlieses ist sehr gering, vorzugsweise wenigstens 8 N/m Breite.

Das von dem Siebband 8 abgegebene Vlies 2 wird vor Eintritt in den Spalt zwischen den beiden Kalanderrollen 6.1 und 6.2 von oben und unten erneut befeuchtet (Befeuchtungsvorrichtung 5).

Zwischen den Kalanderrollen 6.1 und 6.2 wird das zunächst lockere Vlies einem Raster von punktförmigen Druckbereichen ausgesetzt, in denen die regellos liegenden Fasern unter hohem Druck aufeinander gedrückt werden, so daß eine innige, sich nach Aufhebung des Druckes nicht-lösende Fusion der Faserkörper erfolgt und eine Faserstoffbahn 100 mit einem Prägemuster erzeugt wird. Die Walzenanordnung kann auch ach als Pixel-Walzen bezeichnet werden.

Dabei wird vermieden, daß das Fasermaterial verkohlt oder karbonisiert. Offensichtlich ist der Druck aber so hoch angesetzt, daß quasi ein Verschmelzen der die Faser ausmachenden Stoffe, das heißt Cellulose und ein Rest an Lignin, und der anderen Stoffen eintritt, wobei eine so innige Verbundenheit eintritt, daß praktisch eine über eine reine Adhäsion hinausgehende Verbindung erzeugt wird. Die losen Cellulose- oder Zellstofffasern werden mit punktuell hohem Druck und durch Drängen der Fasern in alle vorhandenen Freiräume miteinander verbunden, zusätzlich verklebt und verfilzt und ergeben eine insgesamt sehr feste Faserstoffbahn.

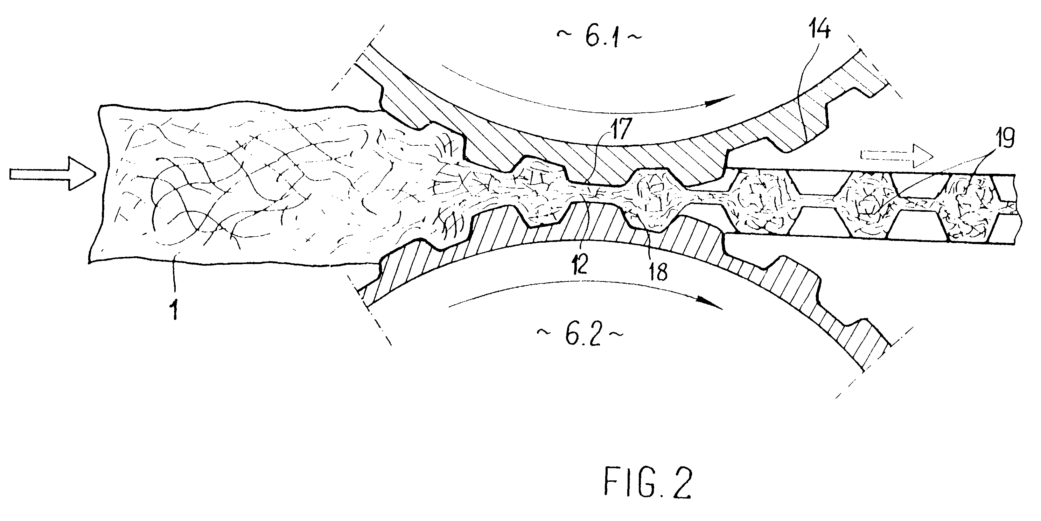

Die Rollen 6.1 und 6.2 werden bei normaler Zimmertemperatur, das heißt zwischen 18 und 25°C betrieben, wobei allerdings nicht ausgeschlossen wird, daß die Rollen auch beheizt werden können oder aber in den punktförmigen Druckbereichen auch punktuell eine höhere Temperatur aufgrund der hohen mechanischen Arbeit erreichen können. Der auf die Cellulose-Faserlage wirkende Druck in den punktförmigen Druckbereichen 17 (vgl. Fig. 2) liegt vorzugsweise oberhalb von 500 MPa, jedenfalls in einem Bereich von 100 und 600 MPa, bei entsprechendem technologischen Aufwand auch höher.

Es lassen sich beispielsweise mit dem Verfahren Faserstoffbahnen mit einem m²-Gewicht zwischen 50 und 1500 g erzeugen. Die aus den Kalandern heraustretende Faserstoffbahn ist wesentlich reißfester als die Bahn vor dem Eintreten in die Kalanderrollen 6.1 und 6.2. Das Material wird mit einer Breitstreckwalze 9 behandelt. Anschließend wird es mit Hilfe einer Treibwalze 10 auf eine Wickelwalze 11 aufgerollt.

Das zum Einsatz gelangende Material sollte in erster Linie ein in großer Menge zur Verfügung stehendes, preiswertes Massenmaterial sein. Vorzugsweise wird ein fluff pulp gewählt, daß eine Weißheit von 85 bis 89% besitzt, was wiederum bedeutet, daß ein Lignin- und Reststoff-Gehalt von erheblichem Ausmaß noch vorhanden ist. Es hat sich gezeigt, daß derartige Reststoffe das Bindungsverhalten wesentlich verbessern. Völlig ausgebleichte Cellulosen haben erfahrungsgemäß ein schlechteres Bindeverhalten als die vorgenannten weniger reinen Cellulosee. Der Titer sollte auch eine gewisse Länge nicht unterschreiten, da bei allzu kurzen Fasern die Abstände zwischen den punktförmigen Druckbereichen nicht überbrückt werden, so daß sich bei derartigen geringen Titern eine geringere Reißfestigkeit ergibt.

Zugefügte Hilfsstoffe werden ebenfalls nach der erwünschten Reißfestigkeit bemessen. Relativ unkritisch ist die Hinzufügung von sogenannten Superabsorbern, wie sie beispielsweise in der Schrift WO 94/10596 genannt werden. Fluff pulp kann mit 0,5 bis 70 Gew.-% Superabsorbern, vorzugsweise 5 bis 30 Gew.-% Superabsorbern versetzt und anschließend durch die Hochdruck-Kalanderrollen 6 geschickt werden. Die Superabsorber wirken nicht bindend; ein zu hoher Anteil setzt die Reißfestigkeit herab.

Das Hinzufügen von gemahlenen nicht-bindenden anorganischen Stoffen, wie dem Weißpigment Titandioxid, verringert allerdings die Reißfestigkeit, so daß z.B. ein Prozentsatz von 25 Gew.% Titandioxid im allgemeinen nicht überschritten werden sollte. Ähnliches gilt für Füllstoffe wie Kaolin oder Zeolithe.

Wesentlich ist, daß auf Bindemittel, wie sie aus dem Stand der Technik bekannt sind und im allgemeinen auch gefordert werden, praktisch völlig verzichtet werden kann. Hierdurch wird die Recyclingfähigkeit und die Kompostierbarkeit des Produktes wesentlich verbessert. Die Herstellung wird verbillig und erleichtert, da Stationen zum Aufbringen und Abbinden (curing) überhaupt nicht eingesetzt werden müssen. Es soll aber nicht ausgeschlossen werden, daß das fertige Produkt nach Durchlauf der Kalanderwalzen 6.1 und 6.2 mit einem Oberflächen-Finish versehen werden kann oder mit einer Folie auf einer oder beiden Seiten laminiert werden kann.

Fig. 2 zeigt ein Ausführungsbeispiel eines HochdruckBereiches zwischen den beiden Kalanderwalzen 6.1 und 6.2. Wie erkennbar, sind die Walzen auf ihrem Walzenmantel mit in vergrößerter Darstellung versehenen Noppen 14 versehen. Die zahlreichen, über die Walzenmantelfläche verteilten Noppen ergeben bei der fertigen Faserstoffbahn vorzugsweise eine Rasterdichte der punktförmigen Druckbereiche zwischen 1 und 16 pro cm². Die Noppen haben eine Pyramidenstumpf-Form, wobei der Winkel des Noppenmantels gegenüber dem Radius zwischen 10 und 45° liegen sollte. Im Spalt 12, in dem der Druckbereich 17 erzeugt wird, herrscht ein berechneter Druck von etwa 520 MPa, der zu der bereits beschriebenen Fusion der sich im Spalt befindlichen Cellulose-Fasern führt. Auch andere Formen der Druckbereiche, wie Kegelstümpfe, Zylinder oder Quader, sind möglich und werden nach fachmännischem Ermessen entsprechend dem erforderlichen Druck, dem vorliegenden Ausgangsstoof und dem Material der Walzen, den auftretenden Temperaturen und dergl. gewählt.

Die Arbeitsrichtung ist im vorliegenden Fall von links nach rechts. Das fertige Produkt weist demnach fast durchsichtige Fusionsbereiche 18 auf, die sich jeweils mit etwas aufgebauschten, jedoch auch gegenüber dem Eingangsvlies zusammengepreßten lockeren Bereichen 19 abwechseln.

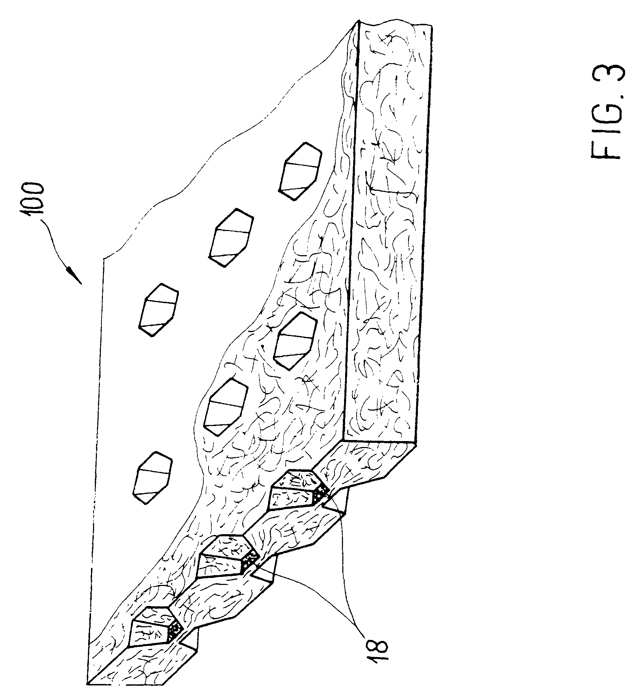

In Fig. 3 ist das fertige Produkt dargestellt, bestehend aus zahlreichen regellosen Cellulose-Fasern, die an Druckbereichen 18 durch Fusion verbunden sind. Das Material selbst hat eine hohe Reißfestigkeit und darüberhinaus eine hohe Absorptionsfähigkeit, die durch Beimischung von Superabsorbern noch erhöht werden kann, so daß es zu Verpackungsmaterial, Hygieneartikeln, Futterstoffen, Polsterfüllstoffen und ähnlichen Produkten verwendet werden kann. Das Material kann aber auch im Baustoffsektor sowie als Ersatz für Papier und Pappe eingesetzt werden. Auch für Servietten, Tampons, Baby-Höschenwindeln, Slipeinlagen, Damenbinden und Inkontinenzartikel lassen sich die vorgenannten Produkte verwenden.

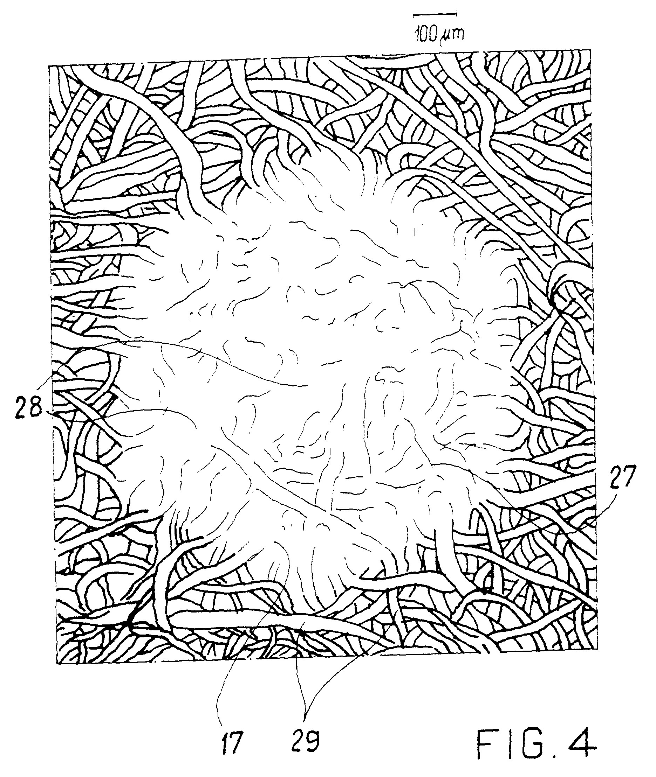

Figur 4 zeigt in vergrößerter Darstellung einen Druckbereich 17 in einer Elektronenmikroskop-Aufnahme. Der Druckbereich hat im vorliegenden Fall eine sechseckige Gestalt, die durch das Einfahren eines Noppen 14 in das Vlies hervorgerufen wird. Der im vorliegenden Fall angewandte Druck beträgt 190 MPa (= 190 N/mm²). Erkennbar ist, daß die zunächst runden und unbeschädigten Fasern 29 im Druckbereich platt und glatt gepreßt sind. Die auch vorhanden gewesenen Superabsorber-Partikel sind optisch nicht mehr auszumachen, da sie offensichtlich in die Oberfläche hineingepreßt werden. Ein Teil der Bereiche 27 innerhalb des Druckbereiches 17 läßt teilweise noch die Faserstruktur erkennen, während andere Bereiche (28) vorhanden sind, in denen überhaupt keine Faserstruktur mehr zu erkennen ist. Die aufeinader gepreßten Fasern lassen sich nicht mehr voneinander trennen, wenn dies mit einer Seziernadel versucht wird. Es hat demnach eine Fusion, Kompaktierung und Verklebung mit oberflächlicher Verschweißung der Faser- und/oder der Cellulose-Substanz stattgefunden, wobei allerdings der Druck unterhalb der Carbonisierungsgrenze der Fasern 29 gehalten wurde.

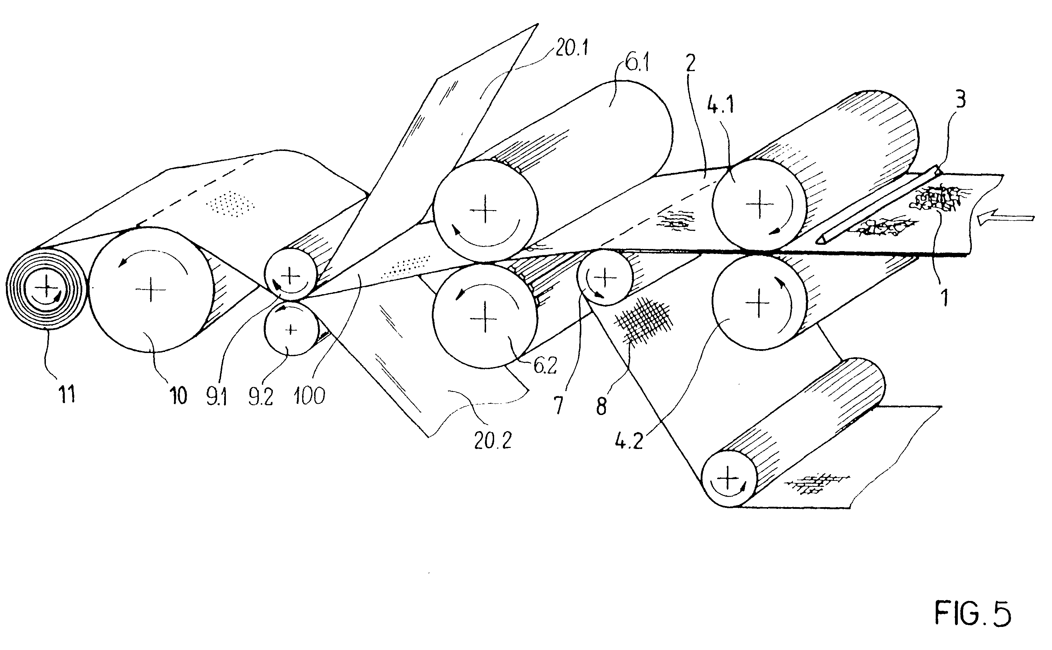

In Fig. 5 ist in schematischer Reihenfolge eine Anordnung von Walzen und Rollen dargestellt, mit denen das Verfahren in einer zweiten Ausführungsform durchgeführt wird. Eine Schicht aus regellosen Fasern 1 von etwa 20 mm Höhe wird auf dem Siebförderband 8 zu einem ersten Kalanderwalzen-Paar 4.1, 4.2 gefördert. Die obere Walze 4.1 hat eine Oberflächentemperatur von etwa 250°C, während die untere unbeheizt ist. Vor Eintritt in den Spalt zwischen den beiden Walzen 4.1 und 4.2 wird die Bahn mit Hilfe einer Befeuchtungsvorrichtung 3 durch Besprühen von der Oberseite her befeuchtet, wobei die Feuchte des Materials danach etwa 5 bis 10 Gew.% ausmacht.

Zwischen den Kalanderwalzen 4.1 und 4.2, wird ein Teil der Feuchte wieder ausgetrieben und die regellose Cellulose-Faserlage zu einem lockerem Vlies mit geringer Dichte und Reißfestigkeit zusammengepreßt.

Zwischen den Kalanderrollen 6.1 und 6.2 wird das zunächst lockere Vlies einem Raster von punktförmigen Druckbereichen ausgesetzt, in denen die regellos liegenden Fasern unter hohem Druck aufeinander gedrückt werden, so daß eine innige, sich nach Aufhebung des Druckes nicht-lösende Fusion der Faserkörper erfolgt und eine Faserstoffbahn 100 mit einem Prägemuster erzeugt wird.

Nach Durchlauf der Kalanderwalzen 6.1 und 6.2 wird die Faserstoffbahn 40 auf beiden Seiten mit Bahnen 20.1 und 20.2 aus textilem, vliesartigem oder folienartigem Material verklebt, verschweißt und/oder mechanisch verbunden. Dazu werden vorgefertigte Beschichtungsbahnen 20.1, 20.2, die - soweit erforderlich - bereits zuvor mit Klebstoffen beschichtet worden sind, von oben und von unten auf die aus dem Kalanderwalzen-paar 6.1, 6.2 austretene Faserstoffbahn geführt und über ein Andruckrollenpaar 9.1, 9.2 mit dieser verbunden. Möglich ist hier auch eine mechanische Verbindung der Beschichtung mit dem Faserstoff über mit Prägeelementen versehene Andruckwalzen 9.1, 9.2. Auch eine Verklebung mittels Heißklebstoff ist möglich. Der Verbund wird mit Hilfe einer Treibwalze 10 auf eine Wickelwalze 11 aufgerollt.

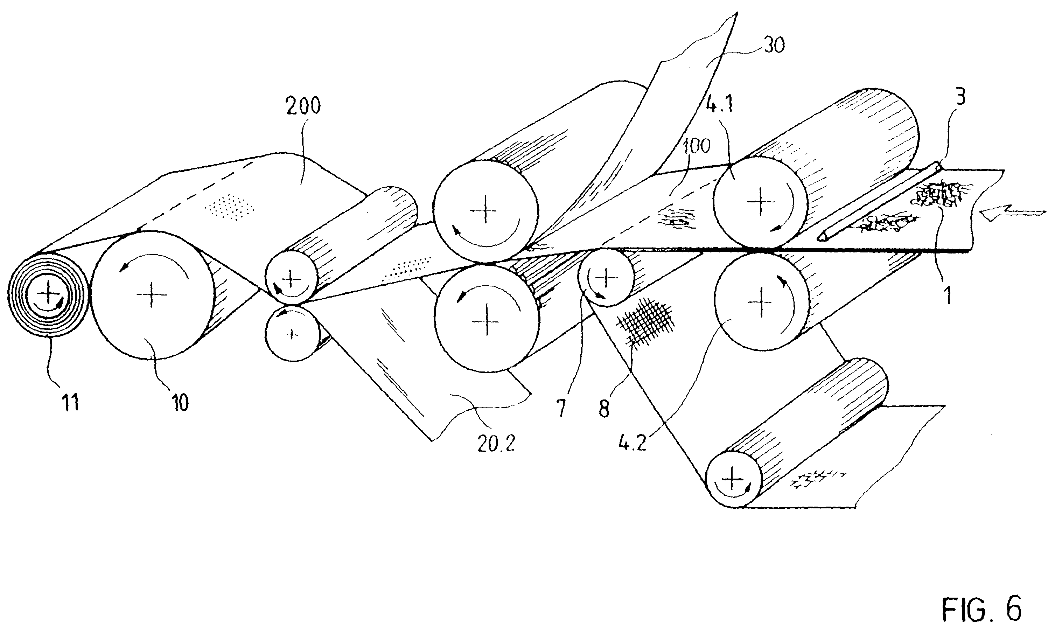

In Fig. 6 ist in schematischer Reihenfolge eine Anordnung von Walzen und Rollen dargestellt, mit denen das Verfahren in einer weiteren Ausführungsform durchgeführt wird. Der Herstellungsprozeß geht aus von Cellulose-Fasern, die als fluff pulp aus trockenem "wood pulp" mit Hilfe von Hammermühlen hergestellt werden.

Ähnlich wie bei Fig. 1 wird eine Schicht aus regellosen Fasern 1 von etwa 20 mm Höhe auf dem Siebförderband 8 zu einem ersten Kalanderwalzen-Paar 4.1., 4.2 gefördert. Die obere Walze 4.1 hat eine Oberflächentemperatur von etwa 180°C, während die untere unbeheizt ist.

Zwischen den Kalanderwalzen 4.1 und 4.2 wird die regellose Cellulose-Faserlage zu einem lockeren Vlies mit geringer Dichte und Reißfestigkeit zusammengepreßt. Das von dem Siebband 8 abgegebene Vlies 2 wird vor Eintritt in den Spalt zwischen den beiden Kalanderrollen 6.1 und 6.2 von oben mit einer dünnen (10 *µ*m) Folie 30 aus PTFE belegt, die zunächst nicht perforiert ist (PTFE = Polyfluorethylen).

Zwischen den Kalanderrollen 6.1 und 6.2 wird das Vlies mit der aufgelegten PTFE-Folie einem Raster von punktförmigen Druckbereichen ausgesetzt, in denen die regellos liegenden Fasern unter hohem Druck aufeinander gedrückt werden, so daß eine innige, sich nach Aufhebung des Druckes nicht-lösende Fusion der Faserkörper erfolgt und eine Faserstoffbahn 100 mit einem Prägemuster erzeugt wird; dabei wird auch die Folie, die relativ wärmebeständig ist, in den Verbund mit einbezogen. Dabei wird vermieden, daß das Faser- oder Folienmaterial verkohlt oder karbonisiert. Eine zusätzliche Bindung wird durch das sinternde oder anschmelzende Folienmaterial erreicht.

Die Rollen 6.1 und 6.2 werden bei normaler Zimmertemperatur, das heißt zwischen 18 und 26° C betrieben, wobei allerdings nicht ausgeschlossen wird, daß die Rollen auch beheizt werden können oder aber in den punktförmigen Druckbereichen auch punktuell eine höhere Temperatur aufgrund der hohen mechanischen Arbeit erreichen können.

Der auf die Cellulose-Faserlage mit der aufgelegten Folie wirkende Druck in den punktförmigen Druckbereichen 17 (vgl. Fig. 4) liegt vorzugsweise oberhalb von 300 bis 400 MPa. Nach Durchlauf der Kalanderwalzen 6.1 und 6.2 ist die Faserstoffbahn auf einer Seite mit einer Bahn aus Folie verbunden. Der Verbund wird mit Hilfe einer Treibwalze 10 auf eine Wickelwalze 11 aufgerollt.

Eine weitere Beschichtungsbahn 20.2, die - soweit erforderlich - bereits zuvor mit Klebstoffen beschichtet worden ist, wird von unten auf die aus dem Kalanderwalzenpaar 6.1, 6.2 austretende Bahn geführt und über ein Andruckrollenpaar 9.1, 9.2 mit dieser verbunden werden (vergl. Figur 6). Der Verbund wird mit Hilfe einer Treibwalze 10 auf eine Wickelwalze 11 aufgerollt.

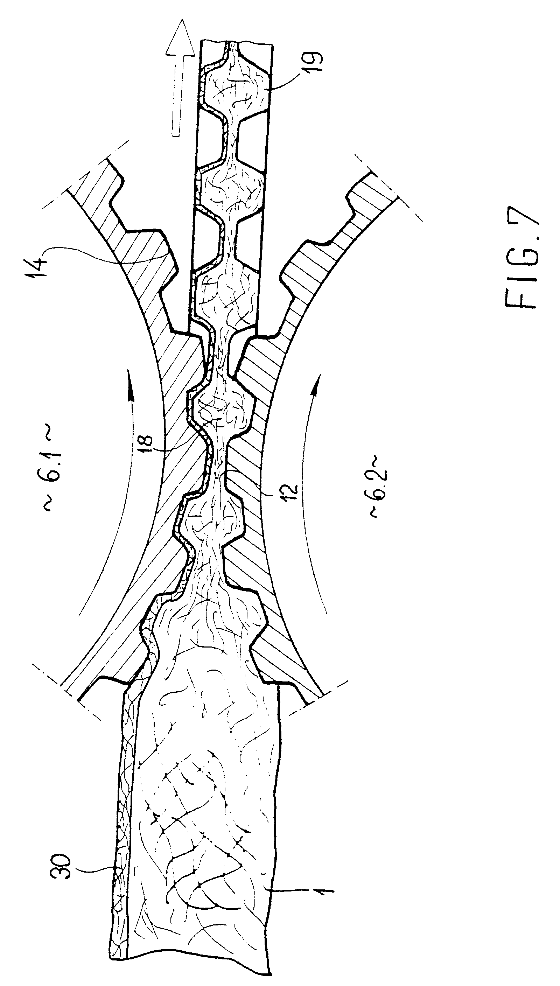

Fig. 7 zeigt ein Ausführungsbeispiel eines Hochdruck-Bereiches zwischen den beiden Kalanderwalzen 6.1 und 6.2. Wie erkennbar, sind die Walzen auf ihrem Walzenmantel mit in vergrößerter Darstellung versehenen Noppen 14 versehen. Die zahlreichen, über die Walzenmantelfläche verteilten Noppen 14 ergeben bei der fertigen Faserstoffbahn vorzugsweise eine Rasterdichte der punktförmigen Druckbereiche zwischen 1 und 16 pro cm₂. Die Noppen haben eine Pyramidenstumpf-Form, wobei der Winkel des Noppenmantels gegenüber dem Radius zwischen 10° und 45° liegen sollte. Im Spalt 12, in dem der Druckbereich 17 erzeugt wird, herrscht ein berechneter Druck von etwa 520 MPa, der zu der bereits beschriebenen Fusion der sich im Spalt befindlichen Cellulose-Fasern führt. Auch andere Formen der Druckbereiche, wie Kegelstümpfe, Zylinder oder Quader, sind möglich und werden nach fachmännischem Ermessen entsprechend dem erforderlichen Druck, dem vorliegenden Ausgangsstoff und dem Material der Walzen, den auftretenden Temperaturen und dgl. gewählt. Dabei kann die Folie 30 mit kalandiert und aufkaschiert werden.

Die Arbeitsrichtung ist bei Fig. 7 von links nach rechts. Das fertige Produkt weist demnach fast durchsichtige Fusionsbereiche 18 auf, die sich jeweils mit etwas aufgebauschten, jedoch auch gegenüber dem Eingangsvlies zusammengepreßten lockeren Bereichen 19 abwechseln.

Die Beschichtungsverfahren werden anhand der nachfolgenden Beispiele näher beschrieben:

### Beispiel 1

Eine Faserstoffbahn 100 (vergl. Fig. 3) wird einseitig mit einer Bahn aus gewebtem textilen Material kombiniert. Die Textilbahn ist an ihrer zur Faserstoffbahn weisenden Fläche mit einem Hotmelt-Klebstoff versehen, so daß nach Durchlaufen der Andruckrollen 9.1, 9.2 ein fester Klebeverbund hergestellt ist. Ein solcher Verbund hat durch den Faserstoff gute wärmeisolierende Wirkung und kann über die gewebte Textilbahn größere mechanische Kräfte aufnehmen.

### Beispiel 2

Die wie anhand der Beschreibung zur Figur 1 bis 3 hergestellte Faserstoffbahn 100 wird an ihrer unbeschichteten Fläche zusätzlich mit einer folienartigen, semipermeablen Klimamembran aus Polytetrafluorethylen über einen Haftkleber verbunden. Die Klimamembran ist wasserabweisend, aber durchlässig für Wasserdampf. Bei der Verwendung als Futtermaterial für hygienische Bekleidungstücke kann der vom Benutzer abgegebene Wasserdampf zunächst vom Fasergewebe aufgenommen und dann über die Klimamembrane abgeleitet werden. Zugleich ist die Faserstoffschicht vor Nässe geschützt.

### Beispiel 3

Eine Vliesbahn 100 wird mit einer Polytetrafluorethylenfolie von 20 *µ*m Dicke zusammengeführt, die einseitig mit einem lösungsmittelfreien Haftkleber beschichtet ist. Über die Kalanderrollen 6.1, 6.2 wird ein Verbund hergestellt. Der Verbund wird auf der unbeschichteten Seite mit einer weiteren Polyethylenfolie vor Eintritt in die Kalanderwalzen 9.1; 9.2 belegt. Durch Nadelwalzen (nicht dargestellt) wird die zweite Polyethylenfolie perforiert. Die beim Perforieren in die Faserstoffbahn eindringenden Folienpartikel bewirken eine mechanische Verankerung zwischen der mit einer ersten Folie beklebten Faserstoffbahn und der zweiten Folie. Es entsteht ein zu einer Oberfläche hin saugfähiges und zur anderen Oberfläche hin flüssigkeitsdichtes Material 200, das sich insbesondere für die Verwendung bei Hygieneartikeln eignet.

Beim Recycling kann die verschmutzte Faserstoffbahn nach dem Abreißen der Foliendeckschichten kompostiert werden. Gegenüber den z.B. bei Wegwerfwindeln eingesetzten Cellulosen mit polymeren Superabsorbern ist bei einem erfindungsgemä&en Verbundwerkstoff eine bessere Umweltverträglichkeit gegeben.

## Patentansprüche

1. Verfahren zur Herstellung einer aus Zellstoff-Fasern aus Zellstoff-Pulpe oder aus Zellstoff-Karton (wood pulp cardbord) bestehenden, ohne Verwendung von zusätzlichen Bindemitteln hergestellten, saugfähigen und rollbaren Faserstoffbahnen (100), die zur Anwendung auf dem Hygienesektor geeignet ist, mit folgenden Verfahrensschritten:
(a) Legen einer regellosen Zellstoff-Faserlage und Vorverdichten unter relativ niedrigem Druck und Erzeugen eines lockeren Vlieses mit geringer Dichte und Reißfestigkeit,
(b) Einführen des lockeren Vlieses in den Spalt eines Kalanderrollen-Paares (6.1,6.2), mit dem ein Muster von punkt- oder linienförmigen Druckbereichen (17) unter einem relativ höherem Druck erzeugt wird, wobei die regellos liegenden Fasern (1) aufeinander gedrückt werden, **dadurch gekennzeichnet, daß**
- der Feuchtigkeitsgehalt bei Einführen des lockeren Vlieses bis zu 5 Gew.-% beträgt,
- die regellos liegenden Fasern (1) im zweiten Kalanderrollen-Paar (6.1,6.2) unter einem Druck im Bereich zwischen 250 und 600 MPa aufeinander gedrückt werden, so daß eine nicht-lösende Fusion der Fasern erfolgt und eine Faserstoffbahn (100) mit einem Prägemuster erzeugt wird,
- und daß die Reißfestigkeit der Faserstoffbahn (100) wenigstens 0,12 kN/m beträgt.

2. Verfahren nach Anspruch 1, dadurch gekennzeichnet, daß eine Faserstoffbahn (100) mit einem m²-Gewicht zwischen 50g und 500 g erzeugt wird.

3. Verfahren nach Anspruch 1 und 2, dadurch gekennzeichnet, daß eine Faserstoffbahn (100) mit einer Rasterdichte der punktförmigen Druckbereiche (17) zwischen 1 und 16 pro cm² erzeugt wird.

4. Verfahren nach wenigstens einem der vorhergehenden Ansprüche, dadurch gekennzeichnet, daß die Fläche der punktförmigen Druckbereiche (17) zwischen 0,05 und 10 mm² liegt.

5. Verfahren nach wenigstens einem der vorhergehenden Ansprüche, dadurch gekennzeichnet, daß die Temperatur des zweiten Kalanderrollen-Paares (6.1,6.2) auf Zimmertemperatur, d.h. zwischen 18 und 25°C, gehalten wird.

6. Verfahren nach wenigstens einem der vorhergehenden Ansprüche, dadurch gekennzeichnet, daß die erste Vorverdichtung bei einer Werkzeugtemperatur von 18 bis 320°C erfolgt.

7. Verfahren nach wenigstens einem der vorhergehenden Ansprüche, dadurch gekennzeichnet, daß als vorverdichtendes Werkzeug ein (erstes) Kalanderrollen-Paar (4.1, 4.2) verwendet wird.

8. Verfahren nach einem der vorhergehenden Ansprüche, dadurch gekennzeichnet, daß als Ausgangsmaterial ein Gemisch von Fasermaterial mit einem Superabsorber verwendet wird, der 0,5 bis 70 Gew.-%, vorzugsweise zwischen 5 bis 30 Gew.-% der Gesamtmasse, ausmacht.

9. Verfahren nach wenigstens einem der vorhergehenden Ansprüche, dadurch gekennzeichnet, daß die als Ausgangsmaterial verwendeten Zellstoff-Fasern (1) einen Weißegrad von 80 bis 90 %, vorzugsweise von 85 bis 89 %, haben.

10. Verfahren nach wenigstens einem der vorhergehenden Ansprüche, dadurch gekennzeichnet, daß als Ausgangsmaterial Zellstoff-Fasern (1) mit einem Restgehalt an Lignin von 0,5 bis 5 Gew.-% verwendet werden.

11. Verfahren nach wenigstens einem der vorhergehenden Ansprüche, dadurch gekennzeichnet, daß als Ausgangsmaterial ein solches mit Hilfs- und Füllstoffen, beispielsweise Titandioxid, Kreide oder Kaolin, verwendet wird.

12. Verfahren nach wenigstens einem der vorhergehenden Ansprüche, dadurch gekennzeichnet, daß im Druckbereich (17) des zweiten Kalanderrollen-Paares (6.1,6.2) der radiale Abstand der Kalanderrollen (6.1,6.2) außerhalb der punkt-förmigen Druckbereiche (17) 1 bis 5 mm beträgt.

13. Verfahren nach wenigstens einem der vorhergehenden Ansprüche, dadurch gekennzeichnet, daß der Spalt (15) im Druckbereich des zweiten Kalanderrollen-Paares (6.1,6.2) zwischen sich gegenüberliegenden punktförmigen Druckbereichen eine lichte Weite von 0,05 bis 1 mm aufweist.

14. Vorrichtung zur Durchführung des Verfahrens nach Anspruch 1 und gegebenenfalls weiteren Ansprüchen 2 bis 13, dadurch gekennzeichnet, daß das zweite Kalanderrollen-Paar aus zwei Kalanderwalzen (6.1,6.2) besteht, die beide mit zahlreichen, über die Walzenmantelflächen verteilten Noppen (14) versehen sind, die von Vertiefungen umgeben sind, die das Mehrfache des Volumens der Noppen (14) aufweisen, und daß die Noppen (14) beider Walzen (6.1,6.2) im Arbeitsspalt gegenüber stehen, wobei in punktförmigen Druckbereichen (17) auf in zwischen den Noppen (14) befindliches lockeres Vlies (2) ein Druck von wenigstens 200 MPa bis Maximal zur Fließgrenze des für die Noppen (14) verwendeten Materials ausübbar ist.

15. Vorrichtung nach Anspruch 14, dadurch gekennzeichnet, daß die Höhe der Noppen (14) gegenüber dem Walzengrund (15) zwischen 0,5 und 5 mm ist.

16. Vorrichtung nach Anspruch 14 oder 15, dadurch gekennzeichnet, daß die Noppen (14) die Form von Pyramidenoder Kegelstümpfen, Quadern und dergleichen haben.

17. Vorrichtung nach Anspruch 16, dadurch gekennzeichnet, daß bei Kegelstumpf- oder Pyramidenform der Winkel des Noppenmantels gegenüber dem Radius zwischen 10 und 45° liegt.

18. Absorbierende Faserstoffmatte zur Herstellung von Hygieneprodukten, hergestellt im Verfahren nach Anspruch 1 und gegebenenfalls weiteren Ansprüchen 1 bis 13.

## Claims

1. A process for the production of absorbent and rollable fibrous material webs (100) consisting of wood pulp fibres, wood pulp, or wood pulp cardboard, without the use of additional binders, such webs being suitable for use in the hygiene sector, with the following process steps:
a) forming a random-orientation wood pulp fibre layer and pre-compression at relatively low pressure and production of a loose web of low density and tearing strength,
b) introduction of the loose web into the nip of a pair of calender rollers (6.1, 6.2), whereby a pattern of punctiform or linear pressure zones (17) is produced at a relatively higher pressure, the random-orientation fibres (1) being pressed one upon the other, **characterised in that:**
- the moisture content on introduction of the loose web is 5% by weight maximum,
- the random-orientation fibres (1) are pressed on one another at a pressure in the range between 250 and 600 MPa in the second pair of calender rollers (6.1, 6.2) so that a permanent fusion of the fibres takes place and a fibrous material web (100) is produced with an embossed pattern,
- and in that the tearing strength of the fibrous material web (100) is at least 0.12 kN/m.

2. A process according to claim 1, characterised in that a fibrous material web (100) is produced with a weight per m² of between 50 g and 500 g.

3. A process according to claims 1 and 2, characterised in that a fibrous material web (100) is produced with a raster density of the punctiform pressure zones (17) of between 1 and 16 per cm².

4. A process according to at least one of the preceding claims, characterised in that the area of the punctiform pressure zones (17) is between 0.05 and 10 mm².

5. A process according to at least one of the preceding claims, characterised in that the temperature of the second pair of calender rollers (6.1, 6.2) is kept at room temperature, i.e. between 18 and 25°C.

6. A process according to at least one of the preceding claims, characterised in that the first pre-compression takes place at a tool temperature of 18 to 320°C.

7. A process according to at least one of the preceding claims, characterised in that the precompressing tool used is a (first) pair of calender rollers (4.1, 4.2).

8. A process according to any one of the preceding claims, characterised in that the starting material used is a mixture of fibrous material with a superabsorber making up 0.5 to 70% by weight, preferably between 5 and 30% by weight, of the total mass.

9. A process according to at least one of the preceding claims, characterised in that the wood pulp fibres (1) used as starting material have a degree of whiteness of 80 to 90%, preferably 85 to 89%.

10. A process according to at least one of the preceding claims, characterised in that the starting material used is wood pulp fibres (1) having a residual lignin content of 0.5 to 5% by weight.

11. A process according to at least one of the preceding claims, characterised in that the starting material used contains adjuvants and fillers, for example titanium dioxide, chalk or kaolin.

12. A process according to at least one of the preceding claims, characterised in that in the pressure zone (17) of the second pair of calender rollers (6.1, 6.2) the radial distance between the calender rollers (6.1, 6.2) outside the punctiform pressure zones (17) is 1 to 5 mm.

13. A process according to at least one of the preceding claims, characterised in that the nip (15) in the pressure zone of the second pair of calender rollers (6.1, 6.2) has a clearance of 0.05 to 1 mm between opposite punctiform pressure zones.

14. Apparatus for performing the process according to claim 1 and where applicable further claims 2 to 13, characterised in that the second pair of calender rollers consists of two calender rollers (6.1, 6.2) both of which have numerous projections (14) distributed over the outer surfaces of the rollers and surrounded by recesses having a volume which is a multiple of the volume of the projections (14) and in that the projections (14) of the two rollers (6.1, 6.2) are opposite one another in the working nip, a pressure of at least 200 MPa to a maximum equivalent to the yield point of the material used for the projections (14) being adapted to be exerted in punctiform pressure zones (17) on loose web (2) situated between the projections (14).

15. Apparatus according to claim 14, characterised in that the height of the projections (14) relative to the roll base (15) is between 0.5 and 5 mm.

16. Apparatus according to claim 14 or 15, characterised in that the projections (14) have the shape of truncated pyramids or truncated cones, parallelepipeds and the like.

17. Apparatus according to claim 16, characterised in that in the case of the truncated cone or pyramid shape the angle of the projection outer surface to the radius is between 10 and 45°.

18. An absorbent fibrous material mat for the production of hygienic products, made by the process according to claim 1 and where applicable further claims 1 to 13.

## Revendications

1. Procédé de fabrication d'une bande (100) de matière fibreuse absorbante et enroulable, constituée de fibres de cellulose, de pulpe de cellulose ou de carton-bois (wood pulp cardbord selon le terme anglo-saxon) et fabriquée sans employer aucun liant additionnel, qui est apte à être utilisée dans le domaine des articles d'hygiène, comprenant les étapes consistant à:
(a) poser une couche irrégulière de fibres de cellulose, la comprimer sous une pression relativement basse et engendrer une nappe ou matelas de fibres lâche, de faible densité et de faible résistance à l'arrachage,
(b) introduire le matelas lâche de fibres dans l'interstice d'une paire de rouleaux de calandrage (6.1, 6.2) au moyen de laquelle un motif de zones de compression ponctuelles ou linéaires (17) est engendré sous une pression relativement plus élevée, ce qui comprime les unes sur les autres les fibres posées irrégulièrement (1), caractérisé en ce que
- la teneur en humidité peut atteindre 5% en poids lors de l'introduction du matelas lâche de fibres;
- les fibres posées irrégulièrement (1) sont comprimées les unes sur les autres dans une deuxième paire de rouleaux de calandrage (6.1, 6.2) sous une pression comprise dans une plage de 250 à 600 MPa, ce qui provoque une fusion indécollable des fibres et produit une bande (100) de matière de fibres à motif gaufré, et
en que la résistance à l'arrachement de la bande (100) de matière fibreuse est au moins de 0,12 kN/m.

2. Procédé selon la revendication 1, caractérisé en ce que le poids par m² de la bande (100) de matière fibreuse produite est compris entre 50 g à 500 g.

3. Procédé selon la revendication 1 ou 2, caractérisé en ce que la densité du réseau des zones de compression ponctuelles (17) dans la bande (100) de matière fibreuse produite est comprise entre 1 et 16 par cm².

4. Procédé selon au moins l'une quelconque des revendications précédentes, caractérisé en ce que la surface des zones de compression ponctuelles (17) est comprise entre 0,05 et 10 mm².

5. Procédé selon au moins l'une quelconque des revendications précédentes, caractérisé en ce que la température de la deuxième paire de rouleaux de calandrage (6.1, 6.2) est maintenue à température ambiante, c'est-à-dire entre 18 et 25°C.

6. Procédé selon au moins l'une quelconque des revendications précédentes, caractérisé en ce que la première précompression est effectuée à une température d'outillage comprise entre 18 et 320°C.

7. Procédé selon au moins l'une quelconque des revendications précédentes, caractérisé en ce qu'une (première) paire de rouleaux de calandrage (4.1, 4.2) est employée comme outillage de précompression.

8. Procédé selon l'une quelconque des revendications précédentes, caractérisé en ce que la matière de départ est un mélange de matière fibreuse et d'un super-absorbant qui constitue 0,5 à 70% en poids, de préférence entre 5 et 30% en poids, de la masse totale.

9. Procédé selon au moins l'une quelconque des revendications précédentes, caractérisé en ce que la qualité du blanc des fibres de cellulose (1) employées comme matière de départ est comprise entre 80 et 90%, de préférence entre 85 et 89%.

10. Procédé selon au moins l'une quelconque des revendications précédentes, caractérisé en ce que la teneur résiduelle en lignine des fibres de cellulose (1) employées comme matière de départ est comprise entre 0,5 et 5%.

11. Procédé selon au moins l'une quelconque des revendications précédentes, caractérisé en ce que la matière de départ comprend des additifs et des charges, par exemple du dioxyde de titane, de la craie ou du kaolin.

12. Procédé selon au moins l'une quelconque des revendications précédentes, caractérisé en ce que la distance radiale des rouleaux de calandrage (6.1, 6.2) dans la zone de compression (17) de la deuxième paire (6.1, 6.2) de rouleaux de calandrage est de 1 à 5 mm en dehors des zones de compression ponctuelles (17).

13. Procédé selon au moins l'une quelconque des revendications précédentes, caractérisé en ce que la largeur libre de l'interstice (15) entre des zones de compression ponctuelles opposées dans la zone de compression de la deuxième paire (6.1, 6.2) de rouleaux de calandrage est de 0,05 à 1 mm.

14. Dispositif de mise en oeuvre du procédé selon la revendication 1 et éventuellement certaines des revendications 2 à 13, caractérisé en ce que la deuxième paire de rouleaux de calandrage se compose de deux cylindres de calandrage (6.1, 6.2) comportant tous deux de nombreux boutons (14) qui sont répartis sur les surfaces d'enveloppe des cylindres et sont entourés par des cavités dont le volume est un multiple de celui des boutons (14), et en ce que les boutons (14) des deux cylindres (6.1, 6.2) sont tournés les uns vers les autres dans l'interstice de travail, une pression égale au moins à 200 MPa et au plus à la limite de fluage de la matière utilisée pour les boutons (14) pouvant être exercée sur le matelas lâche (2) de fibres qui se trouve entre les boutons (14).

15. Dispositif selon la revendication 14, caractérisé en ce que la hauteur des boutons (14) par rapport à la base (15) des cylindres est comprise entre 0,5 et 5 mm.

16. Dispositif selon la revendication 14 ou 15, caractérisé en ce que la configuration des boutons (14) est pyramidale, tronconique, parallélépipédique ou similaire.

17. Dispositif selon la revendication 16, caractérisé en ce que l'angle entre l'enveloppe des boutons et le rayon est compris entre 10 et 45° dans le cas d'une configuration tronconique ou pyramidale.

18. Natte de matière fibreuse absorbante pour la fabrication d'articles d'hygiène, fabriquée selon le procédé de la revendication 1 et éventuellement certaines des revendications 2 à 13.
